# FASCICULE DE BREVET EUROPEEN

(11) **EP 0 868 155 B1**
(45) Date de publication et mention de la délivrance du brevet: **11.08.2004**
(21) Numéro de dépôt: 96934897.8
(22) Date de dépôt: 11.10.1996
(51) Int. Cl.: A61F 2/06

(54) **GAINE EXPANSIBLE CINTRABLE A USAGE CHIRURGICAL POUR DILATATION DE CONDUITS PHYSIOLOGIQUES**
EXPANDIERBARER, BIEGBARER STENT FÜR CHIRURGISCHE VERWENDUNG ZUM AUSDEHNEN EINES KÖRPERLUMENS
FLEXIBLE EXPANDABLE TUBE FOR SURGICAL DILATING OF PHYSIOLOGICAL DUCTS

(43) Date de publication de la demande: 07.10.1998
(73) Titulaire: Fouere, Alain, 13008 Marseille (FR)
(72) Inventeur: Fouere, Alain, 13008 Marseille (FR)
(74) Mandataire: Roman, Michel
(86) Numéro de dépôt international: PCT/FR1996/001590
(87) Numéro de publication internationale: WO 1998/016172

(56) Documents cités:
- EP-A- 0 364 787
- EP-A- 0 709 067
- WO-A-96/18359
- US-A- 5 449 373

## Description

La présente invention a pour objet une gaine expansible cintrable à usage chirurgical pour dilatation de conduits physiologiques.

Elle est avant tout destinée à être mise en place dans une artère ou une veine pour empêcher localement son écrasement afin de permettre le passage du sang, de manière à limiter les troubles circulatoires causés par une pression sanguine trop élevée, ou les risques d'infarctus provoqués par l'oblitération d'un vaisseau, par exemple du myocarde.

Ce type de prothèse est en soi connu et utilisé depuis plusieurs années. Il en existe plusieurs modèles, fabriqués en faisant appel à des techniques variées.

A titre d'exemple, on peut citer le brevet européen N° EP 221 570 déposé par M. Julio Palmaz, qui décrit une prothèse vasculaire expansible comprenant un organe de forme tubulaire à paroi mince formée par une pluralité d'éléments allongés s'intersectant l'un avec l'autre, cet élément tubulaire présentant un diamètre permettant de le faire passer à l'intérieur de la section de passage du conduit récepteur, ce diamètre pouvant être augmenté par l'application, par l'intérieur de l'élément tubulaire, d'une force s'étendant radialement vers l'extérieur, certains des éléments allongés étant déformés de façon définitive par la force exercée radialement vers l'extérieur pour amener l'élément tubulaire à son diamètre expansé, ce par quoi l'élément tubulaire peut dilater la section de passage du conduit et demeurer dans cet état.

Le brevet international N° WO 92 06 734 déposé par M. Ho Young SONG fait état d'une prothèse destinée à élargir le passage de conduits physiologiques, constituée d'une structure cylindrique auto-extensible radialement réalisée en fil métallique soudé et incluant une série de sections droites rendues solidaires les unes des autres par des éléments de liaison périphériques longitudinaux disposés en quinconce ou en diagonale, certains de ces éléments comportant des "barbes" anti-migration, faisant saillie vers l'extérieur. La paroi externe de la prothèse est recouverte d'un filet assurant l'étanchéité de l'ensemble. La mise en place est effectuée en comprimant la prothèse pour l'introduire dans un tube monté à l'extrémité d'un cathéter, l'élasticité du matériau constitutif assurant son extension radiale dès qu'elle est libérée.

Ce dispositif présente un certain nombre d'inconvénients. Sa fabrication est complexe et onéreuse, et l'auto-extension ne permet pas de contrôler la pression qu'il exerce sur la face interne du conduit.

Lorsque la lésion s'étend sur une partie importante du vaisseau à traiter, il est généralement nécessaire de mettre en place plusieurs gaines extensibles à la suite les unes des autres, en raison des sinuosités du conduit, ce qui conduit à laisser les paries présentant une courbe sans protection contre l'écrasement. A notre connaissance, aucune gaine existante ne permet de résoudre ce problème.

La demande de brevet international N° PCT/FR 95/01 460 déposée par l'auteur de la présente invention décrit une gaine tubulaire - ou manchon - formée d'une portion de cylindre creux dont la paroi, réalisée en matériau semi-rigide malléable, est découpée de manière à déterminer des éléments annulaires et longitudinaux jouant le rôle de "mailles" et agencés de manière à permettre à ladite gaine de s'étendre radialement sous l'effet d'une pression interne et de garder la forme ainsi obtenue. Ce manchon peut être constitué de plusieurs éléments articulés entre eux grâce à une bande longitudinale solidaire de deux éléments contigus, des crochets, éventuellement amovibles, pouvant être prévus pour bloquer une ou plusieurs articulations. Les articulations peuvent également être obtenues au moyen d'un filet ou d'un film souple permettant au dispositif d'être placé dans une portion courbée d'un vaisseau.

Ces techniques, tout en permettant de placer un manchon extensible dans une zone de vaisseau comportant une courbe, ne permettent pas d'éviter l'écrasement de la zone cintrée.

Le dispositif selon la présente invention, défini dans la revendication 1, a précisément pour objectif de proposer une gaine expansible destinée à assurer la dilatation de vaisseaux, apte à exercer une pression radiale contre la paroi interne de ceux-ci, sur toute la longueur du dispositif, aussi bien sur les parties cintrées que sur les parties droites.

La présente invention concerne également un procédé d'obtention d'un tel dispositif, comme revendiqué dans la revendication 5.

Il est constitué d'un tube de section circulaire à paroi mince réalisé en métal malléable de manière à pouvoir s'étendre radialement sous l'effet d'une pression interne et garder la forme ainsi obtenue, ce tube étant composé d'au moins deux éléments non cintrables à structure en treillis expansible reliés l'un à l'autre par une zone cintrable formées de barres longitudinales en zigzag, l'ensemble étant agencé pour assurer une force radiale d'expansion contre la paroi interne du vaisseau d'un bout à l'autre de la gaine.

Sur les dessins annexés, donnés à titre d'exemples non limitatifs de formes de réalisation de l'objet de la demande:
la figures 1 est une vue développée d'un mode de réalisation de gaines expansibles selon l'invention comportant deux éléments non cintrables
et la figure 2 représente en coupe axiale longitudinale une gaine expansible montée sur un ballonnet gonflable.

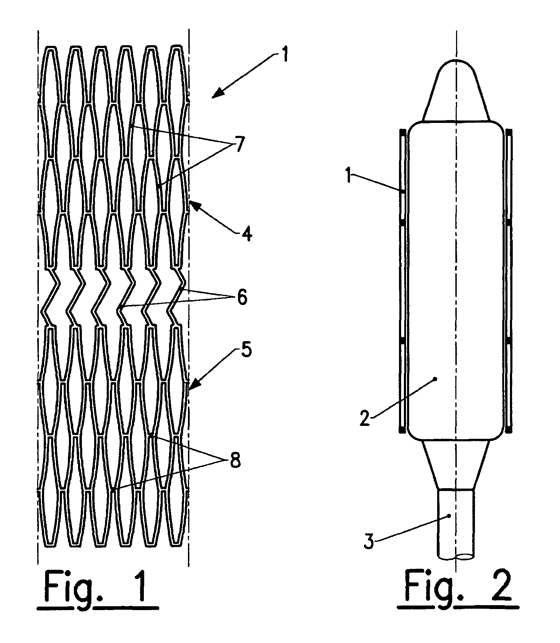

Le dispositif, figures 1 et 2, est constitué d'une gaine tubulaire 1 formée d'une portion de cylindre creux de section circulaire, et dont la paroi consiste en un treillis ou grillage réalisé en matériau semi-rigide malléable agencé de manière à permettre à ladite gaine de s'étendre radialement sous l'effet d'une pression interne et de garder la forme ainsi obtenue. La pression interne est provoquée par exemple au moyen d'un ballonnet gonflable 2 monté à l'extrémité d'un cathéter 3 selon une technique connue (figure 2).

L'ensemble est composé de deux éléments de maintien non cintrables 4, 5 présentant une structure en treillis expansible et reliés par une série de barres 6 périphériques longitudinales ayant chacune la forme d'une ligne brisée en zigzag et agencées pour permettre un cintrage de la gaine 1 au droit de ces barres tout en assurant une force radiale d'expansion d'un bout à l'autre du dispositif.

La gaine 1 sera de préférence fabriquée sous forme expansée, puis "écrasée" sur le ballonnet gonflable 2, et réalisée en une seule pièce par électro-érosion laser à partir d'un bloc de métal tel que l'acier inoxydable ou le tantale, ou par gravure ou usinage laser d'un tube métallique à paroi mince.

Le treillis constituant les éléments non cintrables 4, 5 sera constitué d'éléments longitudinaux 7 reliés entre eux par des éléments annulaires 8 disposés en quinconce formant ainsi des mailles (figure 1) permettant une extension ajustable en fonction de la pression appliquée. Les éléments longitudinaux 7 seront sinueux et formeront des méandres opposés deux à deux, deux éléments longitudinaux voisins se rapprochant puis s'éloignant graduellement, et ainsi de suite (figures 1).

Pour les vaisseaux sanguins, les éléments longitudinaux 7 et annulaires 8, ainsi que les barres en zigzag auront avantageusement une largeur et une épaisseur voisines de 0,1 mm, la longueur des mailles du treillis étant de l'ordre de 4 mm.

Pour certaines applications, la gaine 1 pourra comporter des moyens d'accrochage intégrés, dirigés radialement vers l'extérieur de façon à s'enfoncer dans la paroi interne du vaisseau sous l'effet de la pression de l'élément gonflable 2, empêchant ainsi toutes possibilités de déplacement du dispositif.

Afin d'éviter que l'endothélium des vaisseaux de pénétrer à l'intérieur de la gaine expansible 1, à travers les mailles constituant sa paroi, celle-ci sera avantageusement recouverte d'un film souple en matériau synthétique tel que polyuréthanne, silicone ou polyester.

Pour faciliter les opérations de mise en place et éventuellement de retrait, la gaine 1 pourra comporter des repères radio-opaques consistant en points ou bandes longitudinales ou annulaires visibles aux rayons X, et constitués de couches d'un métal lourd tel que le tantale, le titane ou l'or déposé par voie électrolytique. Ces couches pourront avoir une épaisseur voisine de 0,1 mm.

La gaine 1 peut avoir une longueur quelconque et être constituée d'un nombre quelconques d'éléments non cintrables 4, 5 reliés deux à deux par des barres périphériques 6 en zigzag.

Le positionnement des divers éléments constitutifs donne à l'objet de l'invention un maximum d'effets utiles qui n'avaient pas été, à ce jour, obtenus par des dispositifs similaires.

## Revendications

1. Gaine expansible cintrable à usage chirurgical pour dilatation de conduits physiologiques, destinée en particulier à être mise en place dans une artère ou une veine pour empêcher localement son écrasement afin de permettre le passage du sang, de manière à limiter les troubles circulatoires causés par une pression sanguine trop élevée, ou les risques d'infarctus provoqués par l'oblitération d'un vaisseau, formée d'un tube de section circulaire à paroi mince apte à s'étendre radialement sous l'effet d'une pression interne et à garder la forme ainsi obtenue et constitué d'au moins deux éléments non cintrables (4, 5) présentant une structure en treillis expansible et reliés l'un à l'autre,
**caractérisée en ce qu'**elle est réalisée en une seule pièce par gravure ou usinage laser d'un tube métallique à paroi mince et **en ce que** les éléments non cintrables (4, 5) sont reliés l'un à l'autre par une série de barres périphériques (6) longitudinales ayant chacune la forme d'une ligne brisée en zigzag et agencées pour permettre un cintrage de la gaine (1) au droit desdites barres tout en assurant une force radiale d'expansion sur la paroi interne du conduit sur toute la longueur de ladite gaine, le treillis constituant les éléments non cintrables résultant de la phase de gravure ou d'usinage (4, 5) étant constitué d'éléments longitudinaux (7) sinueux curvilignes formant des méandres opposés deux à deux.

2. Gaine expansible selon la revendication 1, **se caractérisant par le fait qu'**elle est destinée à des vaisseaux sanguins et que les éléments longitudinaux (7) et annulaires (8), ainsi que les barres périphériques (6) en zigzag ont une largeur et une épaisseur voisines de 0,1 mm, la longueur des mailles du treillis étant de l'ordre de 4 mm.

3. Gaine expansible selon l'une quelconque des revendications précédentes, **se caractérisant par le fait que** sa paroi est recouverte d'un film souple en matériau synthétique tel que polyuréthanne, silicone ou polyester, de manière à empêcher l'endothélium du conduit traité de pénétrer à l'intérieur de la gaine à travers les mailles constituant ladite paroi.

4. Gaine expansible selon l'une quelconque des revendications précédentes, **se caractérisant par le fait qu'**elle comporte des repères radio-opaques consistant en points ou bandes longitudinales ou annulaires visibles aux rayons X constitués de couches d'un métal lourd tel que le tantale, le titane ou l'or déposé par voie électrolytique.

5. Procédé de réalisation d'une gaine expansible conforme aux revendications précédentes, **se caractérisant par le fait que** ladite gaine est fabriquée sous forme expansée, puis "écrasée" sur un ballonnet gonflable (2) destiné à être monté à l'extrémité d'un cathéter (3).

## Claims

1. Bendable expansible sheath for surgical use in the dilation of physiological ducts, in particular for placing in an artery or a vein to prevent its narrowing locally, allow blood to pass, and limit the circulatory problems due to high blood pressure, or the risks of heart attacks caused by the blocking of a vessel, made of a circular section tube with thin walls able to spread radially under the effect of an internal pressure and to retain the shape thus obtained and consisting of at least two non-bendable elements (4, 5) with an expansible lattice structure and connected one to the other,
**characterized in that** it is made of a single part by laser etching or machining of a thin-walled metal tube and **in that** the non-bendable elements (4, 5) are connected one to the other by a series of longitudinal peripheral bars (6) having each one a broken-line zigzag shape and arranged to allow bending of sheath (1) at the level of the aforesaid bars while ensuring a radial expansion force is applied on the inner wall of the duct over the entire length of the aforementioned sheath, the lattice constituting non-bendable elements (4, 5) resulting from the etching or machining phase made up of sinuous curvilinear longitudinal elements (7) forming opposite meanders two by two.

2. Expansible sheath according to claim 1, **characterized in that** it is intended for blood vessels and that longitudinal (7) and annular elements (8), as well as the peripheral zigzag bars (6) have a width and a thickness close to 0.1 mm, the length of the lattice meshes being about 4 mm.

3. Expansible sheath according to any of the preceding claims, **characterized in that** its wall is covered with a flexible film in synthetic material such as polyurethane, silicone or polyester, so as to prevent the endothelium of the treated tube from penetrating inside the sheath through the meshes constituting the said wall.

4. Expansible sheath according to any of the preceding claims, **characterized in that** it comprises radio-opaque reference points or longitudinal strips or rings visible with x-rays, consisting of layers of a heavy metal such as tantalum, titanium or gold deposited by electrolysis.

5. Method of executing an expansible sheath in conformity with the aforesaid claims, **characterized in that** the aforementioned sheath is manufactured in expanded form, then "pressed" onto a small inflatable balloon (2) to be fitted on the end of a catheter (3).

## Patentansprüche

1. Dehnbarer und biegsamer Schlauch für chirurgische Zwecke zur Erweiterung physiologischer Kanäle, insbesondere bestimmt zum Einsetzen in eine Arterie oder eine Vene, um örtlich deren Verschließen zu verhindern, d.h. um den Blutdurchfluss zu gewährleisten, so dass Kreislaufstörungen durch zu hohen Blutdruck vermieden werden, desgleichen die Gefahr eines Herzinfarkts, der durch Verschluss eines Gefäßes hervorgerufen wird, wobei der Schlauch aus einem Rohr mit kreisförmigem Querschnitt und dünner Wandung besteht, das sich unter der Einwirkung des Innendrucks radial ausdehnen kann und die so erhaltene Form beibehält und dass aus mindestens zwei nicht biegsamen Elementen (4, 5) besteht, die eine dehnbare Gitterstruktur besitzen und miteinander verbunden sind,
**gekennzeichnet dadurch, dass** er durch Gravur oder Laserbearbeitung aus einem dünnwandigen Metallrohr gefertigt wurde und die nicht biegsamen Elemente (4, 5) durch eine Reihe von in Längsrichtung auf dem Umfang angeordneten Stäben (6) verbunden sind, welche die Form einer gezackten Linie besitzen und so angeordnet sind, dass sie ein Biegen des Schlauchs (1) an diesen Stäben erlauben und gleichzeitig über die gesamte Länge des Schlauchs eine radiale Expansionskraft auf die innere Wandung eines physiologischen Kanals ausüben, wobei das Gitter, das die nicht biegbaren, bei der Gravur oder materialabtragenden Bearbeitung entstehenden Elemente (4, 5) darstellt, aus in Längsrichtung (7) angeordneten, sinusförmigen, Mäander bildenden Elementen besteht, die sich paarweise gegenüber liegen.

2. Dehnbarer Schlauch gemäß Anspruch 1, **gekennzeichnet dadurch, dass** er für Blutgefässe bestimmt ist und die in Längsrichtung angeordneten (7) und ringförmigen (8) Elemente sowie die zickzackförmig über dem Umfang verteilten Stäbe (6) eine Breite und Dicke von etwa 0,1 mm besitzen und die Länge der Maschen dieses Gitters in der Größenordnung von 4 mm liegt.

3. Dehnbarer Schlauch nach einem der vorstehenden Ansprüche, **gekennzeichnet dadurch, dass** seine Wandung mit einem weichen Kunststofffilm beschichtet ist, der z.B. aus Polyurethan, Silikon oder Polyester sein kann, so dass das Endothel des behandelten Gefäßes daran gehindert wird, durch die Maschen, aus denen die Wandung besteht, in das Innere des Schlauchs vorzudringen.

4. Dehnbarer Schlauch gemäß einem der vorstehenden Ansprüche, **gekennzeichnet dadurch, dass** er für Röntgenstrahlen undurchdringliche Markierungen besitzt, die aus punkt- oder bandförmigen Schichten eines Schwermetalls, zum Beispiel auf elektrolytischem Wege aufgebrachtem Tantal, Titan oder Gold, bestehen, ringförmig oder in Längsrichtung verlaufen können und im Röntgenbild sichtbar sind.

5. Verfahren zur Herstellung eines dehnbaren Schlauchs gemäß den obenstehenden Ansprüchen, **gekennzeichnet dadurch, dass** der Schlauch in gedehnter Form gefertigt und anschließend auf einem aufblasbaren Ballon (2) zusammengequetscht wird, der auf das Ende eines Katheters (3) zu montieren ist.
